# EUROPEAN PATENT SPECIFICATION

(11) **EP 4 461 784 B1**
(45) Date of publication and mention of the grant of the patent: **24.09.2025**
(21) Application number: 23211561.8
(22) Date of filing: 22.11.2023
(51) Int. Cl.: C10G 3/00, C10G 29/20, C10G 57/00, C10G 65/12, C10G 69/04, C10G 69/12, C07C 2/66, C07C 5/333, C07C 7/163

(54) **HEAT AND HYDROGEN INTEGRATION IN PROCESS FOR RENEWABLE ALKYLBENZENE PRODUCTS**
WÄRME- UND WASSERSTOFFINTEGRATION IN VERFAHREN FÜR ERNEUERBARE ALKYLBENZOLPRODUKTE
INTÉGRATION DE CHALEUR ET D'HYDROGÈNE DANS UN PROCÉDÉ POUR DES PRODUITS D'ALKYLBENZÈNE RENOUVELABLES

(30) Priority: 30.05.2023 US 202363504880 P
(43) Date of publication of application: 13.11.2024
(73) Proprietor: UOP LLC, Des Plaines, Illinois 60017-5017 (US)
(72) Inventor: DO, Phuong T.M., Charlotte, 28202 (US); WEXLER, James T., Charlotte, 28202 (US); DU, Hai, Charlotte, 28202 (US); JERORO, Eseoghene, Charlotte, 28202 (US); WHITCHURCH, Patrick C., Charlotte, 28202 (US); FICHTL, Geoffrey W., Charlotte, 28202 (US)
(74) Representative: Boult Wade Tennant LLP

(56) References cited:
- US-A1- 2013 079 570
- US-A1- 2013 338 410
- US-A1- 2014 364 355
- US-A1- 2018 179 124

## Description

### BACKGROUND

Linear alkylbenzenes are organic compounds with the formula C₆H₅CₙH₂ₙ₊₁. While the alkyl carbon number, "n" can have any practical value, detergent manufacturers desire that alkylbenzenes have alkyl carbon number in the range of 9 to 16 and preferably in the range of 9 to 14. These specific ranges are often required when the alkylbenzenes are used as intermediates in the production of surfactants for detergents. The alkyl carbon number in the range of 9 to 14 falls in line with the specifications of the detergents industry.

Because the surfactants created from alkylbenzenes are biodegradable, the production of alkylbenzenes has grown rapidly since their initial uses in detergent production in the 1960s. The linearity of the paraffin chain in the alkylbenzenes is key to the material's biodegradability and effectiveness as a detergent. A major factor in the final linearity of the alkylbenzenes is the linearity of the paraffin component.

While detergents made utilizing alkylbenzene-based surfactants are biodegradable, previous processes for creating alkylbenzenes are not based on renewable sources. Specifically, alkylbenzenes are currently produced from kerosene refined from crude extracted from the earth. Due to the growing environmental prejudice against fossil fuel extraction and economic concerns over exhausting fossil fuel deposits, there may be support for using an alternate source for biodegradable surfactants in detergents and in other industries.

US2014/364355A1 discloses a process for the production of alkylbenzenes from natural oils comprising deoxygenation, dehydrogenation and alkylation.

Accordingly, it is desirable to provide linear alkylbenzenes with a high degree of linearity that are made from biorenewable sources instead of being extracted from the earth. Further, it is desirable to provide renewable linear alkylbenzenes from easily processed triglycerides and fatty acids from vegetable, animal, nut, and/or seed oils. These nC9 to nC14 intermediate products are useful in eventually making linear alkylbenzene types of detergents through additional process steps. It is further desirable that the resulting nC9 to nC14 paraffins are linear products with a minimum of branched isomer products. It is also desirable to reduce the amount of energy used in the process, as well as the amount of hydrogen required.

### BRIEF DESCRIPTION OF THE DRAWINGS

Fig. 1 is a schematic view of one embodiment of a process for producing alkylbenzenes from natural oils incorporating hydrogen and heat integration according to the present invention.
Fig. 2 is a plot of the mass-% normal paraffins versus deoxygenation temperature in accordance with Example 2.

### DETAILED DESCRIPTION

The invention is in accordance with the appended claims. The present invention relates to a process for producing alkylbenzenes from natural oils, such as vegetable, animal, nut, seed oils, and/or triglyceride-containing oils. Natural oils are not based on kerosene or other fossil fuels. Natural oils include those derived from plant or algal material, animal fats, nut, and/or seed oils, and/or triglyceride-containing oils, and are often referred to as renewable oils. Natural oils typically comprise triglycerides, free fatty acids, or combinations thereof. Natural oils include, but are not limited to, Arachis oil (peanut oil; groundnut oil), Babassu oil, Coconut oil, Cottonseed oil, Grapeseed oil, Maize oil (corn oil), Mustard seed oil, Palm kernel oil, Palm oil, Palm olein (the liquid fraction derived from the fractionation of palm oil), Palm stearin (the high-melting fraction derived from the fractionation of palm oil), Rapeseed oil, Rapeseed oil - low erucic acid (low erucic acid turnip rape oil; low erucic acid colza oil; canola oil), Safflowerseed oil (safflower oil; carthamus oil; kurdee oil), Safflowerseed oil - high oleic acid (high oleic acid safflower oil; high oleic acid carthamus oil; high oleic acid kurdee oil), Sesameseed oil (sesame oil; gingelly oil; benne oil; ben oil; till oil; tillie oil) , Soya bean oil (soybean oil), Sunflowerseed oil (sunflower oil), and Sunflowerseed oil - high oleic acid (high oleic acid sunflower oil).

The process for making alkylbenzenes from triglycerides according to the present invention involves the deoxygenation of the triglycerides to form paraffins. The paraffins are separated (by fractionation, distillation, and the like) into a C9 to C14 stream comprising C9 to C14 paraffins and a C14+ stream comprising C14+ (i.e., containing carbon chains from C15 to C28) paraffins. The C14+ stream is sent to a separate linear selective cracking unit to crack the C14+ paraffins; the cracked paraffins are fractionated into a first stream comprising the C9 to C14 normal and lightly branched paraffins and a second stream comprising isoparaffins. Optionally, contaminants, including but not limited to, comprise sulfur compounds, or nitrogen compounds, or phosphorous compounds, or oxygenates, or aromatics or combinations thereof, are removed from the first stream, or the C9 to C14 stream, or both. The decontaminated stream is dehydrogenated to form olefins, di-olefins, and aromatics. The di-olefins are selectively hydrogenated to form additional olefins, and the aromatics are separated and removed forming an aromatics stream comprising the aromatics and a mono-olefin stream comprising the mono-olefins. Benzene is alkylated with the olefins, and the alkylation effluent comprises alkylbenzenes and benzene. The alkylbenzenes are then isolated.

This general process can be improved by providing the heat produced in various processes to other processes which require the addition of heat. For example, the heat generated in dehydrogenating the natural oils can be used to reduce the amount of heat needed in one or more of the linear selective cracking unit, the dehydrogenation unit, the alkylation unit. Furthermore, the heat present in certain streams can be used to heat other streams. For example, the heat present in the dehydrogenated stream, or the alkylation effluent stream, or both can be used to heat one or more of the paraffin stream, or the C9 to C14 stream, or the C14+ stream, or the first stream, or the second stream.

In addition, the process can incorporate appropriate hydrogen recycle. The dehydrogenation process produces hydrogen which can be recycled to the linear selective cracking unit to be used in cracking the C14+ paraffins.

The process will be described in more detail below.

To limit catalyst deactivation, the feed is treated to remove sulfur contamination before hydrodeoxygenation. Otherwise, sulfur accumulates on the catalyst and leads to deactivation. A high temperature hydrogen treatment was shown to recover some of the lost activity. The degree of hydrodeoxygenation can affect the selectivity to each of the normal paraffins in the 9 to 14 carbon range. A large degree of hydrodeoxygenation can bias the hydrodeoxygenated composition largely in favor of normal dodecane and normal decane to the detriment of normal undecane and normal tridecane. A small degree of hydrodeoxygenation can bias the hydrodeoxygenated composition in favor of normal undecane and normal tridecane to the detriment of normal dodecane and normal decane..

The hydrodeoxygenation reactor temperatures are kept low, less than 343°C (650°F) for typical biorenewable feedstocks and less than 304°C (580°F) for feedstocks with higher free fatty acid (FFA) concentration to avoid polymerization of olefins found in FFA. Generally, hydrodeoxygenation reactor pressure of 700 kPa (100 psig) to 21 MPa (3000 psig) are suitable.

The linearity of the alkylbenzene product is mostly dependent on the linearity of the paraffins used to alkylate the benzene. It is a common rule of thumb by those skilled in the art that the linearity of a paraffin feed drops by 5-7 mass % after dehydrogenation and alkylation. Therefore, paraffin with 97 mass % linearity (or alternatively 3 mass % isoparaffin) would result in an alkylbenzene product with linearity around 90-92 mass %. This sets the requirement for paraffin linearity 5-7 mass % higher than the specification for the alkylbenzene product. Typically the linearity of the paraffin product is measured by UOP 621, UOP411, or UOP732 standard test method available from ASTM. Linear alkylbenzenes may be analyzed using ASTM Standard Test Method D4337.

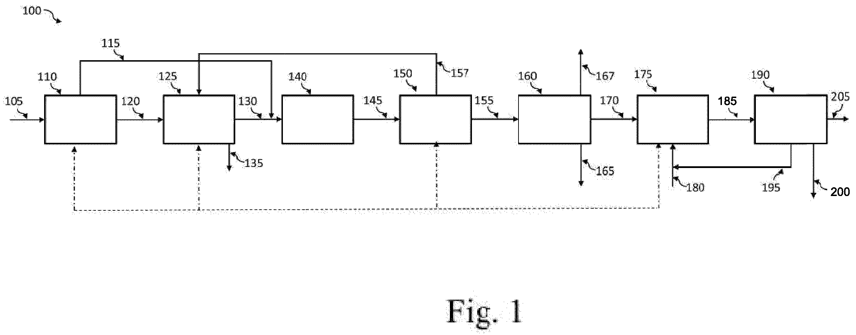

In Fig. 1, an exemplary system 100 for producing an alkylbenzene product from a natural oil feed is illustrated.

In the illustrated embodiment, the natural oil feed 105 is delivered to a deoxygenation unit 110 which also receives a hydrogen feed (not shown). In the deoxygenation unit 110, the fatty acids in the natural oil feed 105 are deoxygenated and converted into normal paraffins. Structurally, triglycerides are formed by three, typically different, fatty acid molecules that are bonded together with a glycerol bridge. The glycerol molecule includes three hydroxyl groups (HO--) and each fatty acid molecule has a carboxyl group (COOH). In triglycerides, the hydroxyl groups of the glycerol join the carboxyl groups of the fatty acids to form ester bonds. Therefore, during deoxygenation, the fatty acids are freed from the triglyceride structure and are converted into normal paraffins. The glycerol is converted into propane, and the oxygen in the hydroxyl and carboxyl groups is converted into water, carbon dioxide, or carbon monoxide. The deoxygenation reaction for fatty acids and triglycerides are respectively illustrated as:

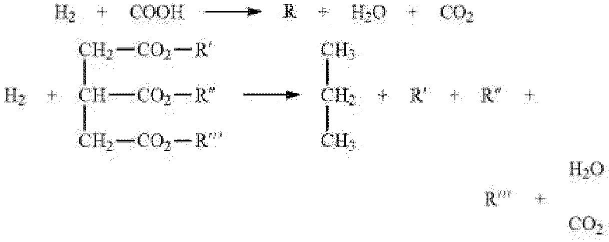

During the deoxygenation reaction, the length of a paraffin chain Rⁿ created will vary by a value of one depending on the exact reaction pathway. It is understood that deoxygenation includes at least one of hydrodeoxygenation, decarboxylation, and decarbonylation, or any combination thereof. For instance, if carbon dioxide is formed, then the chain will have one fewer carbon than the fatty acid source. If water is formed, then the chain will match the length of the fatty acid source.

Operating conditions for the deoxygenating unit include pressures in the range of from 1724 to 5516 kPa (250 to 800 psig) and temperatures in the range of from 274°C to 371°C (525°F to 700°F) in one embodiment, from 274°C to 338°C (525°F to 640°F) in another embodiment and from 274°C to 310°C (525°F to 590°F) in another embodiment. Catalysts may include those containing one or more of Ni, Mo, Co, P, such as Ni--Mo, Ni--Mo--P, NiCo--Mo, or Co--Mo, on alumina, silica, titania, zirconia, and mixtures thereof. Suitable hydrogen to hydrocarbon mole ratios include from 42.48 to 283.17 (1500 to 10,000), from 113.27 to 254.85 (4000 to 9000), and from 141.58 to 226.53 (5000-8000) m³ per barrel of feedstock (standard cubic feet per barrel of feedstock (scf/B)). Suitable space velocities include 0.2-3.0 hr⁻¹ LHSV. Conditions are selected to minimize cracking or isomerizing the paraffins.

The deoxygenated product containing normal paraffins, water, carbon dioxide, carbon monoxide, and propane is fractionated into a C9 to C14 stream 115 and a C14+ stream 120. The separation may be performed in a multi-stage fractionation unit, distillation system or similar known apparatus. In any event, the separator removes the water, carbon dioxide, carbon monoxide, and propane from the deoxygenated product. A naphtha stream of paraffins with carbon chain lengths of C₅ to C₉ (not shown) may also be formed.

The C14+ stream 120 is sent to the linear selective cracking unit 125 where it is selectively cracked to form a first stream 130 comprising normal or lightly branched C9 to C14 paraffins and a second stream 135 comprising isoparaffins. The linear selective cracking takes place in a separate unit, rather than in the bottom bed of a first stage hydrocracking reactor because sulfur and nitrogen contaminants from the first stage can poison a metal-based hydrocracking catalyst. The C14+ paraffins are selectively cracked over the C9 to C14 due to higher absorption energy.

Selection of particular metal catalysts, including noble metals (such as ruthenium and platinum), and nickel can produce a much higher yield of normal paraffins with 9-14 carbons than previous processes. Suitable catalysts include, but are not limited to, Ru/ZrO₂, a Pt-Al₂O₃, Ni-alumina, or a NiOₓ/clay. With these catalysts, the C14+ stream is able to generate linear cracking products without significant amounts of branched isomer production.

Of the preferred catalysts, the Ru catalyst exhibits much higher activity and per-pass nC9 to nC14 yield than the other catalysts. Under the optimized reaction conditions, it also produces very small amounts of methane and isomerized product. This has been found to be the best catalyst for such chemical transformation process. The Pt-Al2O3 catalyst can produce even lower methane yield than the Ru based catalyst with slightly less linear product yield.

The C9 to C14 stream 115 from the deoxygenation unit 110 and the first stream 130 from the linear selective cracking unit 125 are sent to a decontamination unit 140. The decontamination unit 140 removes contaminants in an adsorption separation system from the C9 to C14 paraffins in the C9 to C14 stream 115 and the first stream 130. The contaminants include, but are not limited to, sulfur compounds, or nitrogen compounds, or phosphorous compounds, or oxygenates, or aromatics or combinations thereof.

The decontaminated stream 145 is sent to a dehydrogenation unit 150 where hydrogen is removed to produce a dehydrogenated stream 155 comprising mono-olefins, di-olefins, and aromatics. In the dehydrogenation unit 150, the paraffins are dehydrogenated into mono-olefins of the same carbon numbers as the paraffins. Typically, dehydrogenation occurs through known catalytic processes, such as the commercially popular Pacol process. Di-olefins (i.e., dienes) and aromatics are also produced as an undesired result of the dehydrogenation reactions as expressed in the following equations:
Mono-olefin formation:

   CₓH₂ₓ₊₂→ CₓH₂ₓ + H₂
Di-olefin formation:

   CₓH₂ₓ → CₓH₂ₓ₋₂ + H₂
Aromatic formation:

   CₓH₂ₓ₋₂ → CₓH₂ₓ₋₆ + 2H₂

Operating conditions for the dehydrogenation unit 150 include space velocities from 5 to 50 LHSV and from 20 to 32 LHSV; pressures from 34 kPa (g) to 345 kPa (g) (5 psig to 50 psig) and from 103 kPa (g) to 172 kPa (g) (15 psig to 25 psig); temperatures from 400°C to 500°C and from 440°C to 490°C, and hydrogen to hydrocarbon mole ratios from 1-12 and from 3-7. An example of a suitable catalyst is a Pt on alumina catalyst where platinum is attenuated with an attenuator metal. Another suitable catalyst is described in U.S. Pat. No. 6,177,381. The dehydrogenation unit 150 may be operated dry or with water injection up to 2000 mass-ppm water. Hydrogen can be recycled to the deoxygenation unit upstream.

Hydrogen is also produced in the dehydrogenation reaction. Hydrogen stream 157 is recycled to the linear selective cracking unit 125 to provide hydrogen for the cracking step.

The dehydrogenated stream 155 is sent to a selective hydrogenation unit 160, such as a DeFine reactor, where at least a portion of the di-olefins are hydrogenated to form additional mono-olefins. As a result, the mono-olefin stream 170 has an increased mono-olefin concentration compared to the dehydrogenated stream 155. The aromatics are separated and removed as aromatics stream 165. A light end stream 167 containing any lights, such as butane, propane, ethane and methane, that resulted from cracking or other reactions during upstream processing can also removed.

The mono-olefin stream 170 comprising mono-olefins is sent to the alkylation unit 175 along with a benzene stream 180. The benzene is alkylated with the mono-olefins to form alkylbenzene. The alkylation unit 175 contains a catalyst, such as a solid acid catalyst, that supports alkylation of the benzene with the mono-olefins. Fluorinated silica-alumina, hydrogen fluoride (HF), aluminum chloride (AlCl₃), zeolitic, and ionic liquid catalysts are examples of major catalysts in commercial use for the alkylation of benzene with linear mono-olefins and may be used in the alkylation unit 175. As a result of alkylation, alkylbenzene, typically called linear alkylbenzene (LAB), is formed according to the reaction:

C₆H₆ + CₓH₂ₓ → C₆H₅CₓH₂ₓ₊₁

Suitable operating conditions for the alkylation unit 175 include space velocities from 1 to 10 LHSV, pressures to maintain liquid phase operation, such as 2068 kPa (g) to 4137 kPa (g) (300 psig to 600 psig), temperatures in the range of from 80°C to 180°C and 120°C to 170°C, benzene to olefin mole ratios of 3 to 40 and 8 to 35.

Surplus amounts of benzene are supplied to the alkylation unit 175 to achieve high degree of desired alkylation. Therefore, the alkylation effluent 185 exiting the alkylation unit 175 contains alkylbenzene and unreacted benzene. Further the alkylation effluent 185 may also include some unreacted paraffins. The alkylation effluent 185 is passed to a benzene separation unit 190, such as a fractionation column, for separating the unreacted benzene and paraffins from the alkylation effluent 185. The unreacted benzene exits the benzene separation unit 190 in a benzene recycle stream 195 that may be sent back into the alkylation unit 175 to maintain the desired benzene/olefin ratio (e.g., 1-50) to reduce the volume of fresh benzene needed. The fresh benzene requirement (i.e., the net benzene) is determined by the net olefin to the alkylation unit A paraffin stream 200 can also be separated out and recycled to the dehydrogenation unit 150.

As a result of the post-alkylation separation processes, the linear alkylbenzene product 205 is isolated. It is noted that such separation processes are not necessary in all embodiments in order to isolate the linear alkylbenzene product 205.

The linear alkylbenzene product 205 is a linear alkylbenzene product comprising: alkylbenzenes having the formula C₆H₅CₙH₂ₙ₊₁ wherein n is from 9 to 14. In some embodiments, at least 80 mass % of the alkylbenzenes have linear alkyl groups, or at least 90 mass %.

The linear alkylbenzene may be sulfonated to provide a linear alkylbenzene sulfonate product comprising: alkylbenzene sulfonate compounds having the formula CₙH₂ₙ₊₁C₆H₄SO₃H wherein n is from 10 to 14, or wherein n is from 11 to 13.

As used herein, the term "separator" means a vessel which has an inlet and at least an overhead vapor outlet and a bottoms liquid outlet and may also have an aqueous stream outlet from a boot. A flash drum is a type of separator which may be in downstream communication with a separator which may be operated at higher pressure. The term "communication" means that fluid flow is operatively permitted between enumerated components, which may be characterized as "fluid communication". The term "downstream communication" means that at least a portion of fluid flowing to the subject in downstream communication may operatively flow from the object with which it fluidly communicates.

The term "column" means a distillation column or columns for separating one or more components of different volatilities. Unless otherwise indicated, each column includes a condenser on an overhead of the column to condense and reflux a portion of an overhead stream back to the top of the column and a reboiler at a bottom of the column to vaporize and send a portion of a bottoms stream back to the bottom of the column. Feeds to the columns may be preheated. The top pressure is the pressure of the overhead vapor at the vapor outlet of the column. The bottom temperature is the liquid bottom outlet temperature. Unless indicated otherwise, overhead lines and bottoms lines refer to the net lines from the column downstream of any reflux or reboil take-off to the column. Stripper columns may omit a reboiler at a bottom of the column and instead provide heating requirements and separation impetus from a fluidized inert media such as steam.

As used herein, the term "a component-rich stream" or "a component stream" means that the stream coming out of a vessel has a greater concentration of the component than the feed to the vessel. As used herein, the term "a component-lean stream" means that the lean stream coming out of a vessel has a smaller concentration of the component than the feed to the vessel.

### EXAMPLES

### Example 1

A coconut oil feed was deoxygenated, to form paraffins, dehydrogenated to form mono-olefins, and benzene was alkylated with the mono-olefins to form an alkylbenzene product with a modern carbon content of 62 mass 96 modern carbon as determined by ASTM D6866 as compared to a theoretical modern carbon content of 66.4 mass %, a bromine number of 1 g Br/per gram sample as determined by UOP standard test method 304, and a linearity of 92 mass %.

### Example 2

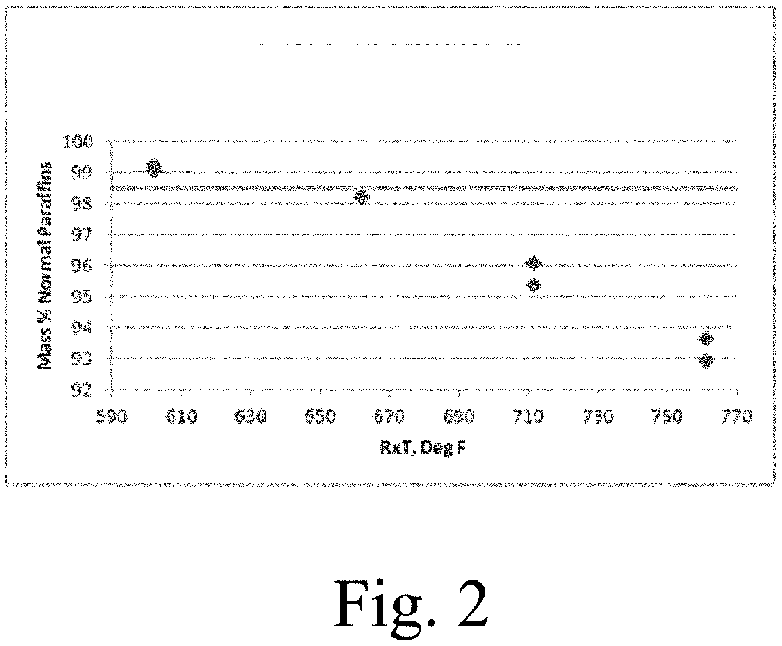

An oil was deoxygenated using a catalyst at a pressure of 3309 KPa (480 psig), H, to bio-oil ratio of 204 m³/B (7200 scf/B) and a LHSV of 1 hr'. During operation, the deoxygenation reaction temperature was increased in steps from 315°C. (600°F) to 34.9°C. (660°F) and then to 377°C. (710°F) and 404°C. (760°F) to monitor the response of linearity in the final product to reaction temperature. The results are shown in Fig. 2 which is a plot of the concentration in mass % of normal C10-C13 paraffins versus reaction temperature. Fig. 2 clearly demonstrates that as the deoxygenation reaction temperature is increased, the concentration of linear paraffins decreases. Controlling the temperature to less than 404°C. (760°F) resulted in greater than 92 mass percent linear paraffins.

Note: Examples 1 and 2 were previously included in US Patent No. 9,079,814 as Examples 3 and 4.

### SPECIFIC EMBODIMENTS

While the following is described in conjunction with specific embodiments, it will be understood that this description is intended to illustrate and not limit the scope of the preceding description and the appended claims.

A first embodiment of the invention is a method for production of a linear alkylbenzene product derived from a natural oil comprising deoxygenating the natural oil to form a paraffin stream comprising paraffins; separating the paraffin stream to form a C9 to C14 stream comprising C9 to C14 paraffins and a C14+ stream comprising C14+ paraffins; linear selective cracking the C14+ stream in a separate linear selective cracking unit under linear selective cracking conditions in the presence of a linear selective cracking catalyst to form a first stream comprising normal or lightly branched C9 to C14 paraffins and a second stream comprising isoparaffins; dehydrogenating the first stream in a dehydrogenation unit to provide a dehydrogenated stream comprising mono-olefins, di-olefins, and aromatics and a hydrogen stream comprising hydrogen; recycling at least a portion of the hydrogen stream to the linear selective cracking unit; selectively hydrogenating the di-olefins in the dehydrogenated stream to form additional mono-olefins, and separating and removing the aromatics from the mono-olefins to form an aromatics stream comprising the aromatics and a mono-olefins stream comprising the mono-olefins; alkylating benzene with the mono-olefins in an alkylation unit under alkylation conditions to provide an alkylation effluent comprising alkylbenzenes and benzene; isolating the alkylbenzenes to provide the alkylbenzene product derived from the natural oil; and heating exchanging the paraffin stream, or the C9 to C14 stream, or the C14+ stream, or the first stream, or the second stream, or combinations thereof with the dehydrogenated stream, or the alkylation effluent stream, or both; or providing heat from deoxygenating the natural oil to the linear selective cracking unit, or the dehydrogenation unit, or the alkylation unit, or combinations thereof; or both. An embodiment of the invention is one, any or all of prior embodiments in this paragraph up through the first embodiment in this paragraph further comprising removing contaminants from the first stream, or the C9 to C14 stream, or both to form a decontaminated stream wherein the contaminates comprise sulfur compounds, or nitrogen compounds, or phosphorous compounds, or aromatics, or oxygenates, or fatty acids, or fatty esters,. or combinations thereof before dehydrogenating the first stream. An embodiment of the invention is one, any or all of prior embodiments in this paragraph up through the first embodiment in this paragraph wherein the C9 to C14 stream and the first stream are combined before removing the contaminants from the first stream, or the C9 to C14 stream, or both to form the decontaminated stream. An embodiment of the invention is one, any or all of prior embodiments in this paragraph up through the first embodiment in this paragraph wherein the linear selective cracking catalyst comprises ruthenium, platinum, and nickel supported catalyst or mixtures thereof. An embodiment of the invention is one, any or all of prior embodiments in this paragraph up through the first embodiment in this paragraph wherein the linear selective racking conditions comprise a temperature in a range of 290 °C to 455°C, or a pressure in a range of 2.8 MPa to 17.5 MPa, or combinations thereof. An embodiment of the invention is one, any or all of prior embodiments in this paragraph up through the first embodiment in this paragraph wherein the alkylbenzene product comprises alkylbenzenes having C9 to C14 chains. An embodiment of the invention is one, any or all of prior embodiments in this paragraph up through the first embodiment in this paragraph wherein the natural oil is selected from vegetable oils, and animal fats and triglyceride-containing oils.

A second embodiment of the invention is a method for production of a linear alkylbenzene product derived from a natural oil comprising deoxygenating the natural oil to form a paraffin stream comprising paraffins, wherein the natural oil is selected from vegetable oils, and animal fats and triglyceride-containing oils; separating the paraffin stream to form a C9 to C14 stream comprising C9 to C14 paraffins and a C14+ stream comprising C14+ paraffins; linear selective cracking the C14+ stream in a separate linear selective cracking unit under linear selective cracking conditions in the presence of a linear selective cracking catalyst to form a first stream comprising normal or lightly branched C9 to C14 paraffins and a second stream comprising ioparaffins; removing contaminants from the first stream, or the C9 to C14 stream, or both to form a decontaminated stream wherein the contaminates comprise sulfur compounds, or nitrogen compounds, or phosphorous compounds, or aromatics, or oxygenates, or fatty acids, or fatty esters, or combinations thereof; dehydrogenating the decontaminated stream in a dehydrogenation unit to provide a dehydrogenated stream comprising mono-olefins, di-olefins, and aromatics and a hydrogen stream comprising hydrogen; recycling at least a portion of the hydrogen stream to the linear selective cracking unit; selectively hydrogenating the di-olefins in the dehydrogenated stream to form additional mono-olefins, and separating and removing the aromatics from the mono-olefins to form an aromatics stream comprising the aromatics and a mono-olefins stream comprising the mono-olefins; alkylating benzene with the mono-olefins in an alkylation unit under alkylation conditions to provide an alkylation effluent comprising alkylbenzenes and benzene; isolating the alkylbenzenes to provide the alkylbenzene product derived from the natural oil; and heating exchanging the paraffin stream, or the C9 to C14 stream, or the C14+ stream, or the first stream, or the second stream, or combinations thereof with the dehydrogenated stream, or the alkylation effluent stream, or both; or providing heat from deoxygenating the natural oil to the linear selective cracking unit, or the dehydrogenation unit, or the alkylation unit, or combinations thereof; or both. An embodiment of the invention is one, any or all of prior embodiments in this paragraph up through the second embodiment in this paragraph wherein the C9 to C14 stream and the first stream are combined before removing the contaminants from the C9 to C14 stream and the first stream to form the decontaminated stream. An embodiment of the invention is one, any or all of prior embodiments in this paragraph up through the second embodiment in this paragraph wherein the linear selective cracking catalyst comprises ruthenium, platinum, and nickel supported catalyst or mixtures thereof. An embodiment of the invention is one, any or all of prior embodiments in this paragraph up through the second embodiment in this paragraph wherein the linear selective racking conditions comprise a temperature in a range of 290 °C to 455°C, or a pressure in a range of 2.8 MPa to 17.5 MPa, or combinations thereof. An embodiment of the invention is one, any or all of prior embodiments in this paragraph up through the second embodiment in this paragraph wherein the alkylbenzene product comprises alkylbenzenes having C9 to C14 chains.

In the foregoing, all temperatures are set forth in degrees Celsius and, all parts and percentages are by weight, unless otherwise indicated.

## Claims

1. A method for production of a linear alkylbenzene product derived from a natural oil comprising:
deoxygenating the natural oil (105) to form a paraffin stream comprising paraffins;
separating the paraffin stream to form a C9 to C14 stream (115) comprising C9 to C14 paraffins and a C14+ stream (120) comprising C14+ paraffins;
linear selective cracking the C14+ stream (120) in a separate linear selective cracking unit (125) under linear selective cracking conditions in the presence of a linear selective cracking catalyst to form a first stream (130) comprising normal or lightly branched C9 to C14 paraffins and a second stream (135) comprising isoparaffins;
dehydrogenating the first stream (130) in a dehydrogenation unit (150) to provide a dehydrogenated stream (155) comprising mono-olefins, di-olefins, and aromatics and a hydrogen stream (157) comprising hydrogen;
recycling at least a portion of the hydrogen stream (157) to the linear selective cracking unit;
selectively hydrogenating the di-olefins in the dehydrogenated stream (155) to form additional mono-olefins, and separating and removing the aromatics from the mono-olefins to form an aromatics stream (165) comprising the aromatics and a mono-olefins stream (170) comprising the mono-olefins;
alkylating benzene (180) with the mono-olefins in an alkylation unit (175) under alkylation conditions to provide an alkylation effluent (185) comprising alkylbenzenes and benzene;
isolating the alkylbenzenes to provide the alkylbenzene product (205) derived from the natural oil; and
heating exchanging the paraffin stream, or the C9 to C14 stream (115) , or the C14+ stream (120), or the first stream (130), or the second stream (135), or combinations thereof with the dehydrogenated stream (155), or the alkylation effluent stream (185), or both; or providing heat from deoxygenating the natural oil to the linear selective cracking unit (125), or the dehydrogenation unit(150), or the alkylation unit (175), or combinations thereof; or both.

2. The method of claim 1 further comprising:
removing contaminants from the first stream (130), or the C9 to C14 stream (115), or both to form a decontaminated stream (145) wherein the contaminates comprise sulfur compounds, or nitrogen compounds, or phosphorous compounds, or aromatics, or oxygenates, or fatty acids, or fatty esters,. or combinations thereof before dehydrogenating the first stream (130).

3. The method of claim 2 wherein the C9 to C14 stream (115) and the first stream (130) are combined before removing the contaminants from the first stream (130), or the C9 to C14 stream (115), or both to form the decontaminated stream (145).

4. The method of any one of claims 1-2 wherein the linear selective cracking catalyst comprises ruthenium, platinum, and nickel supported catalyst or mixtures thereof.

5. The method of any one of claims 1-2 wherein the linear selective cracking conditions comprise: a temperature in a range of 290 °C to 455°C, or a pressure in a range of 2.8 MPa to 17.5 MPa, or combinations thereof.

6. The method of any one of claims 1-2 wherein the alkylbenzene product (205) comprises alkylbenzenes having C9 to C14 chains.

7. The method of any one of claims 1-2 wherein the natural oil (105) is selected from vegetable oils, and animal fats and triglyceride-containing oils.

## Patentansprüche

1. Verfahren zur Produktion eines linearen Alkylbenzolprodukts, das aus einem natürlichen Öl abgeleitet ist, umfassend:
Desoxidieren des natürlichen Öls (105), um einen Paraffinstrom auszubilden, der Paraffine umfasst;
Trennen des Paraffinstroms, um einen C9-bis-C14-Strom (115), der C9-bis-C14-Paraffine umfasst, und einen C14+-Strom (120), der C14+-Paraffine umfasst, auszubilden;
lineares selektives Cracken des C14+-Stroms (120) in einer separaten lineares-selektives-Cracking-Einheit (125) unter lineares-selektives Cracking-Bedingungen in Anwesenheit eines lineares-selektives-Cracking-Katalysators, um einen ersten Strom (130), der normale oder leicht verzweigte C9-bis-C14-Paraffine umfasst, und einen zweiten Strom (135), der Isoparaffine umfasst, auszubilden;
Dehydrieren des ersten Stroms (130) in einer Dehydrierungseinheit (150), um einen dehydrierten Strom (155), der Mono-Olefine, Di-Olefine und Aromaten umfasst, und einen Wasserstoffstrom (157), der Wasserstoff umfasst, bereitzustellen;
Rückführen von mindestens einem Teil des Wasserstoffstroms (157) zu der lineares-selektives-Cracking-Einheit;
selektives Hydrieren der Di-Olefine in dem dehydrierten Strom (155), um zusätzliche Mono-Olefine auszubilden, und Trennen und Beseitigen der Aromaten von den Mono-Olefinen, um einen Aromatenstrom (165), der die Aromaten umfasst, und einen Mono-Olefinen-Strom (170), der die Mono-Olefine umfasst, auszubilden;
Alkylieren von Benzol (180) mit den Mono-Olefinen in einer Alkylierungseinheit (175) unter Alkylierungsbedingungen, um ein Alkylierungsabwasser (185) bereitzustellen, das Alkylbenzole und Benzol umfasst;
Isolieren der Alkylbenzole, um das Alkylbenzolprodukt (205) bereitzustellen, das von dem natürlichen Öl abgeleitet ist; und
Wärmetauschen des Paraffinstroms oder des C9-bis-C14-Stroms (115) oder des C14+-Stroms (120) oder des ersten Stroms (130) oder des zweiten Stroms (135) oder von Kombinationen derselben mit dem dehydrierten Strom (155) oder dem Alkylierungsabwasserstrom (185) oder mit beiden; oder Bereitstellen von Wärme aus der Desoxidierung des natürlichen Öls an die lineares-selektives-Cracking-Einheit (125) oder an die Dehydrierungseinheit (150) oder an die Alkylierungseinheit (175) oder von Kombinationen derselben oder beidem.

2. Verfahren nach Anspruch 1, ferner umfassend:
Beseitigen von Kontaminationsstoffen aus dem ersten Strom (130) oder dem C9-bis-C14-Strom (115) oder beiden, um einen dekontaminierten Strom (145) auszubilden, wobei die Kontaminationsstoffe Schwefelverbindungen oder Stickstoffverbindungen oder Phosphorverbindungen oder Aromaten oder Oxygenaten oder Fettsäuren oder Fettester oder Kombinationen derselben umfassen, vor dem Dehydrieren des ersten Stroms (130).

3. Verfahren nach Anspruch 2, wobei der C9-bis-C14-Strom (115) und der erste Strom (130) vor dem Beseitigen der Kontaminationsstoffe aus dem ersten Strom (130) oder dem C9-bis-C14-Strom (115) oder aus beiden, um den dekontaminierten Strom (145) auszubilden, kombiniert werden.

4. Verfahren nach einem der Ansprüche 1 bis 2, wobei der lineares-selektives-Cracking-Katalysator einen Ruthenium-, Platin- und Nickel-geträgerten Katalysator oder Mischungen derselben umfasst.

5. Verfahren nach einem der Ansprüche 1 bis 2, wobei die lineares-selektives-Cracking-Bedingungen Folgendes umfassen: eine Temperatur in einem Bereich von 290 °C bis 455 °C oder einen Druck in einem Bereich von 2,8 MPa bis 17,5 MPa oder Kombinationen derselben.

6. Verfahren nach einem der Ansprüche 1 bis 2, wobei das Alkylbenzolprodukt (205) Alkylbenzole umfasst, die C9-bis-C14-Ketten aufweisen.

7. Verfahren nach einem der Ansprüche 1 bis 2, wobei das natürliche Öl (105) ausgewählt ist aus Pflanzenölen und tierischen Fetten und Triglycerid-haltigen Ölen.

## Revendications

1. Procédé de production d'un produit alkylbenzène linéaire dérivé d'une huile naturelle comprenant :
désoxygéner l'huile naturelle (105) pour former un flux de paraffine comprenant des paraffines ;
séparation du flux de paraffines pour former un flux C9 à C14 (115) comprenant des paraffines C9 à C14 et un flux C14+ (120) comprenant des paraffines C14+ ;
craquage sélectif linéaire du flux C14+ (120) dans une unité distincte de craquage sélectif linéaire (125) dans des conditions de craquage sélectif linéaire en présence d'un catalyseur de craquage sélectif linéaire pour former un premier flux (130) comprenant des paraffines C9 à C14 normales ou légèrement ramifiées et un second flux (135) comprenant des isoparaffines ;
déshydrogénation du premier flux (130) dans une unité de déshydrogénation (150) pour fournir un flux déshydrogéné (155) comprenant des mono-oléfines, des di-oléfines et des aromatiques et un flux d'hydrogène (157) comprenant de l'hydrogène ;
recyclage d'au moins une partie du flux d'hydrogène (157) dans l'unité de craquage sélectif linéaire,
hydrogéneration sélective des di-oléfines dans le flux déshydrogéné (155) pour former des mono-oléfines supplémentaires, et séparation et élimination des aromatiques des mono-oléfines pour former un flux d'aromatiques (165) comprenant les aromatiques et un flux de mono-oléfines (170) comprenant les mono-oléfines ;
alkylation du benzène (180) avec les mono-oléfines dans une unité d'alkylation (175) dans des conditions d'alkylation pour obtenir un effluent d'alkylation (185) comprenant des alkylbenzènes et du benzène ;
isolation des alkylbenzènes pour obtenir le produit alkylbenzène (205) dérivé de l'huile naturelle ; et
échange de chaleur entre le flux de paraffine, ou le flux C9 à C14 (115), ou le flux C14+ (120), ou le premier flux (130), ou le second flux (135), ou des combinaisons de ceux-ci, et le flux déshydrogéné (155), ou le flux d'effluent d'alkylation (185), ou les deux ; ou apport de chaleur provenant de la désoxygénation de l'huile naturelle à l'unité de craquage sélectif linéaire (125), ou à l'unité de déshydrogénation (150), ou à l'unité d'alkylation (175), ou à des combinaisons de ceux-ci ; ou les deux.

2. Procédé de la revendication 1 comprend en outre :
élimination des contaminants du premier flux (130), ou du flux C9 à C14 (115), ou des deux, pour former un flux décontaminé (145) dans lequel les contaminants comprennent des composés sulfurés, ou des composés azotés, ou des composés phosphorés, ou des aromatiques, ou des composés oxygénés, ou des acides gras, ou des esters gras, ou des combinaisons de ceux-ci avant de déshydrogéner le premier flux (130).

3. Procédé de la revendication 2 dans lequel le flux C9 à C14 (115) et le premier flux (130) sont combinés avant d'éliminer les contaminants du premier flux (130), ou du flux C9 à C14 (115), ou des deux, pour former le flux décontaminé (145).

4. Procédé de l'une quelconque des revendications 1 à 2, dans lequel le catalyseur de craquage sélectif linéaire comprend un catalyseur à base de ruthénium, de platine et de nickel ou des mélanges de ces éléments.

5. Procédé de l'une quelconque des revendications 1 à 2, dans lequel les conditions de craquage sélectif linéaire comprennent : une température comprise entre 290 °C et 455 °C, ou une pression comprise entre 2,8 MPa et 17,5 MPa, ou des combinaisons de ces deux éléments.

6. Procédé de l'une quelconque des revendications 1 à 2, dans lequel le produit alkylbenzène (205) comprend des alkylbenzènes ayant des chaînes en C9 à C14.

7. Procédé de l'une quelconque des revendications 1 à 2, dans lequel l'huile naturelle (105) est choisie parmi les huiles végétales, les graisses animales et les huiles contenant des triglycérides.
